(19)

Europäisches
Patentamt

European
Patent Office

Office européen
des brevets

(11) **EP 4 067 933 B1**

(12) **EUROPEAN PATENT SPECIFICATION**

(45) Date of publication and mention
of the grant of the patent:
**07.05.2025 Bulletin 2025/19**

(21) Application number: **21166152.5**

(22) Date of filing: **31.03.2021**

(51) International Patent Classification (IPC):
*G01S 7/539* (2006.01)      *G01S 15/96* (2006.01)
*G01S 15/66* (2006.01)

(52) Cooperative Patent Classification (CPC):
**G01S 7/539; G01S 15/66; G01S 15/96**

(54) **FISH SIZE CALCULATION DEVICE AND METHOD**

VORRICHTUNG UND VERFAHREN ZUR BERECHNUNG DER FISCHGRÖSSE

DISPOSITIF ET PROCÉDÉ DE CALCUL DE LA TAILLE DES POISSONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.10.2022 Bulletin 2022/40**

(73) Proprietor: **FURUNO ELECTRIC CO., LTD.
Nishinomiya-City, Hyogo 662-8580 (JP)**

(72) Inventor: **ITO, Masanori
Nishinomiya-city, Hyogo 662-8580 (JP)**

(74) Representative: **Cleveland Scott York
5 Norwich Street
London EC4A 1DR (GB)**

(56) References cited:
**EP-A1- 3 875 988         WO-A1-2017/163904
WO-A1-2020/090287**

- **EGERTON J P ET AL: "Hydroacoustics for the
discovery and quantification of Nassau grouper
(Epinephelus striatus) spawning aggregations",
CORAL REEFS, SPRINGER VERLAG, BERLIN,
DE, vol. 36, no. 2, 18 January 2017 (2017-01-18),
pages 589 - 600, XP036237824, ISSN: 0722-4028,
[retrieved on 20170118], DOI: 10.1007/
S00338-017-1542-4**

**EP 4 067 933 B1**

**Description**

The present disclosure relates to signal processing in a fish finder in an aquaculture environment, and more particularly to fish size calculation using ultrasonic device.

"Hydroacoustics for the discovery and quantification of Nassau grouper spawning aggregations", (Coral Reefs 2017, vol. 36 no. 2), describes a study for estimating fish abundance and biomass of a specific endangered fish species, including using a split-beam echosounder and analysing echo data based on echo integration. To calculate a target strength from individual fish, fish echoes are tracked on a plurality of pings.

WO2017/163904A1 (Furuno Electric Co.) describes an underwater detection apparatus wherein an echo signal, received in response to a ultrasonic signal transmitted into water, is used to detect individual information about an underwater moving body, and information such as moving speed of the body is calculated therefrom.

In a fish farm, fish are raised in fish cages, and in order to check that the fish are raised properly, there is a need to regularly measure the size or the weight of the fish. Also, it is preferable to remotely measure the fish without actually physically touching the fish, in order not to stress or hurt the fish.

As a method to remotely measure the fish, ultrasound is conventionally used by placing a transducer in water in the fish cage and by acquiring echoes of the fish using a conventional fish finder as explained in for example, WO2020/090287. In a typical fish echo signal acquired from such fish finder, where the horizontal axis of the fish echo signal indicates time, *i.e.,* depth, and where the vertical axis indicates strength of the echo, *i.e.,* target strength, a weight of the fish can be calculated by calculating the time difference between two consecutive peaks within the echo signal of the fish. The highest peak indicates an echo received from the swimbladder of the fish whereas the other peaks indicate echoes received from other body parts such as a back of the fish.

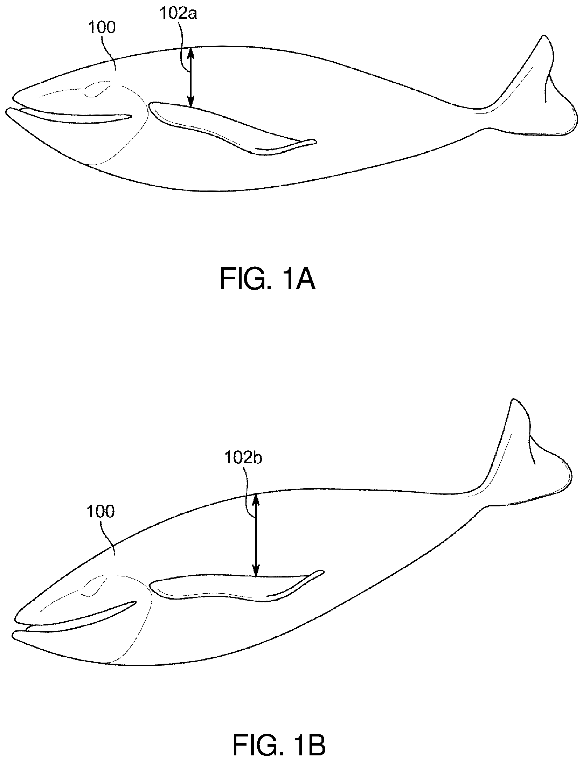

However, the distance, *i.e.,* the time between the swimbladder and the back of the fish may change depending from which direction the ultrasounds are penetrating inside the fish, and thus, an ultrasound incidence angle, *i.e.,* an angle at which the ultrasounds penetrate inside the fish may lead to errors in the fish weight calculation. **FIGs. 1A** and **1B** illustrate such principle. **FIG. 1A** illustrates a fish **100** swimming in a horizontal direction and **FIG. 1B** illustrates the fish **100** swimming in a head-down direction. In these two different swimming directions, if ultrasounds are propagating in a vertical direction, the distance between the swimbladder and the back of the fish when the fish swims in the horizontal direction of **FIG. 1A** is the distance **102a**, whereas the distance between the swimbladder and the back of the fish when the fish swims in the head-down direction of **FIG. 1B** is the distance **102b**. Depending on fish species, the distances **102a** and **102b** may be different.

Hence, there is a need to improve accuracy of the fish weight calculation by taking into consideration, the ultrasound incidence angle of the ultrasounds on the fish, *i.e.,* penetrating direction of the ultrasounds within the fish.

In a first aspect, the present invention provides a target size calculation device that includes a transducer, a fish tracking module, an ultrasound incidence angle calculation module, a distance calculation module, and a fish weight calculation module. The transducer is configured to transmit a transmission wave toward underwater targets and generate an echo signal from a reflection of the transmission wave on the underwater targets. The fish tracking module is configured to track in time a fish among the underwater targets from echo signals resulting from different transmission waves. The ultrasound incidence angle calculation module is configured to calculate, for each position of the tracked fish in time, an ultrasound incidence angle of the transmission wave on the tracked fish based on the echo signals. The distance calculation module is configured to calculate, for each position of the tracked fish in time, a distance between a swimbladder of the tracked fish and a second body part of the tracked fish, different from the swimbladder, from the echo signals of the tracked fish. The fish weight calculation module is configured to calculate a weight of the tracked fish based on the ultrasound incidence angle and the calculated distance.

Additionally, the target size calculation device includes an ultrasound incidence angle-distance histogram calculation module configured to calculate an ultrasound incidence angle vs. distance histogram from the ultrasound incidence angle and the distance calculated for a plurality of tracked fish.

Additionally, or optionally, the target size calculation device includes a peak extraction module configured to extract a first peak and a second peak from the histogram, and calculate a first peak distance of the first peak on the histogram and a second peak distance of the second peak on the histogram.

Additionally, or optionally, the fish weight calculation module is configured to calculate a mean weight of the plurality of tracked fish from the first peak distance and the second peak distance.

In a second aspect, the present invention provides a target size calculation device that includes a transducer, a fish tracking module, an ultrasound incidence angle calculation module, a distance calculation module, and a fish weight calculation module. The transducer is configured to transmit a transmission wave toward underwater targets and generate an echo signal from a reflection of the transmission wave on the underwater targets. The fish tracking module is configured to track in time a fish among the underwater targets from echo signals resulting from different transmission waves. The ultrasound incidence angle calculation module is configured to calculate, for each position of the tracked fish in time, an ultrasound incidence angle of the transmission wave on the tracked fish based on the echo signals. The distance

2

calculation module is configured to calculate, for each position of the tracked fish in time, a distance between a swimbladder of the tracked fish and a second body part of the tracked fish, different from the swimbladder, from the echo signals of the tracked fish. The fish weight calculation module is configured to calculate a weight of the tracked fish based on the ultrasound incidence angle and the calculated distance. The fish weight calculation module is configured to calculate a weight of the tracked fish when a first ultrasound incidence angle of the transmission wave on the tracked fish at a first timing and a second ultrasound incidence angle of the transmission wave on the tracked fish at a second timing different from the first timing are such that a given threshold angle is in-between the first and second ultrasound incidence angles.

The fish weight calculation module calculates the weight based on the distance of the tracked fish in the first ultrasound incidence angle and the distance of the tracked fish in the second ultrasound incidence angle.

Additionally, or optionally, the ultrasound incidence angle calculation module calculates the ultrasound incidence angle by calculating an angle made between a first line that connects two different positions of the tracked fish in time and a second line that connects one of the two different positions and a position of the transducer.

Additionally, or optionally, the ultrasound incidence angle calculation module calculates the ultrasound incidence angle by calculating an angle made between a second line that connects a position of the tracked fish and the transducer and a third line that is vertical line from the transducer toward bottom of the water.

Additionally, or optionally, the ultrasound incidence angle calculation module calculates the ultrasound incidence angle based on a first distance between the transducer and a first position of the tracked fish at a first time instance and a second distance between the transducer and a second position of the tracked fish at a second time instance different from the first time instance, wherein the ultrasound incidence angle calculation module calculates the ultrasound incidence angle based on an assumption that the first and second distances change with time based on a hyperbolic curve.

Additionally, or optionally, the second body part is an outer surface of the tracked fish.

In a third aspect of the present invention, there is provided a target size calculation method that includes transmitting a transmission wave toward underwater targets and generating an echo signal from a reflection of the transmission wave on the underwater targets using a transducer, tracking in time a fish among the underwater targets from echo signals resulting from different transmission waves, calculating for each position of the tracked fish in time, an ultrasound incidence angle of the transmission wave on the tracked fish based on the echo signals, calculating for each position of the tracked fish in time, a distance between a swimbladder of the tracked fish and a second body part of the tracked fish, different from the swimbladder, from the echo signals of the tracked fish, calculating a weight of the tracked fish based on the ultrasound incidence angle and the calculated distance; and calculating an ultrasound incidence angle vs. distance histogram from the ultrasound incidence angle and the distance calculated for a plurality of tracked fish.

In a fourth aspect of the present invention, there is provided a non-transitory computer readable medium having stored thereon computer-executable instructions which, when executed by a device of the first aspect of the present invention, cause the device to execute the method of the third aspect of the present invention.

In a fifth aspect of the present invention, there is provided a target size calculation method, comprising that includes transmitting a transmission wave toward underwater targets and generating an echo signal from a reflection of the transmission wave on the underwater targets using a transducer, tracking in time a fish among the underwater targets from echo signals resulting from different transmission waves, calculating for each position of the tracked fish in time, an ultrasound incidence angle of the transmission wave on the tracked fish based on the echo signals, calculating for each position of the tracked fish in time, a distance between a swimbladder of the tracked fish and a second body part of the tracked fish, different from the swimbladder, from the echo signals of the tracked fish, and calculating a weight of the tracked fish based on the ultrasound incidence angle and the calculated distance, wherein the weight of the tracked fish is calculated when a first ultrasound incidence angle of the transmission wave on the tracked fish at a first timing and a second ultrasound incidence angle of the transmission wave on the tracked fish at a second timing different from the first timing are such that a given threshold angle is in-between the first and second ultrasound incidence angles, and wherein the weight is calculated based on the distance of the tracked fish in the first ultrasound incidence angle and the distance of the tracked fish in the second ultrasound incidence angle.

In a sixth aspect of the present invention there is provided a non-transitory computer readable medium having stored thereon computer-executable instructions which, when executed by the device of the second aspect of the present invention, cause the device to execute the method of the fifth aspect of the present invention.

The problem of not being able to calculate fish weight with enough accuracy with ultrasound is solved by calculating a swimbladder-back of fish distance in different ultrasound incidence angles of the transmission wave on the fish.

These as well as other aspects, advantages, and alternatives will become apparent to those of ordinary skill in the art by reading the following detailed description with reference where appropriate to the accompanying drawings. Further, it should be understood that the description provided in this summary section and elsewhere in this document is intended to illustrate the claimed subject matter by way of example and not by way of limitation.

The illustrated embodiments of the subject matter will be best understood by reference to the drawings, wherein like parts are designated by like numerals throughout. The following description is intended only by way of example, and simply illustrates certain selected embodiments of devices, systems, and processes that are consistent with the subject matter as claimed herein:

**FIG. 1A** illustrates a fish swimming in a horizontal direction;
**FIG. 1B** illustrates a fish swimming in a head-down direction;
**FIG. 2** is a block diagram showing an overall configuration of a target size calculation device, in accordance with a first embodiment of the present invention;
**FIG. 3** is a perspective view showing how a target size calculation device is used in a cage, in accordance with an embodiment of the present disclosure;
**FIG. 4** illustrates an example of an envelope of first, second and third echo signals generated based on first, second and third transmission waves $T_1$, $T_2$ and $T_3$ by the transducer respectively;
**FIG. 5A** illustrates the fish tracked at two positions using two consecutive transmissions $T_{n-1}$ and $T_n$ by the transducer, in accordance with an embodiment of the present disclosure;
**FIG. 5B** illustrates the fish tracked at two positions using two consecutive transmissions $T_{n-1}$ and $T_n$ by the transducer, in accordance with another embodiment of the present disclosure;
**FIG. 5C** illustrates the fish tracked at two positions using two consecutive transmissions $T_{n-1}$ and $T_n$ by the transducer, in accordance with yet another embodiment of the present disclosure;
**FIG. 6** illustrates calculation of a swimbladder-back distance for the tracked fish for a given transmission;
**FIG. 7A** illustrates an ultrasound incidence angle vs swimbladder-back distance histogram, in accordance with an embodiment of the present disclosure;
**FIG. 7B** illustrates a one dimensional swimbladder-back distance histogram, in accordance with an embodiment of the present disclosure;
**FIG. 8** illustrates a graph that shows result of five experiments performed with fish type yellowtail in which mean fish weight has been calculated;
**FIG. 9** is a block diagram showing an overall configuration of a target size calculation device, in accordance with a second embodiment of the present invention;
**FIG. 10** illustrates first and second ultrasound incidence angles of a transmission wave on a tracked fish tracked on first and second transmissions $T_{n-1}$ and $T_n$ by the transducer respectively;
**FIG. 11A** illustrates a top view showing the calculation of the ultrasound incidence angle when the transducer includes a single receiving channel, in accordance with an embodiment of the present disclosure;
**FIG. 11B** illustrates a side view showing the calculation of the ultrasound incidence angle when the transducer includes a single receiving channel, in accordance with an embodiment of the present disclosure;
**FIG. 12** illustrates various echo signals generated at different time instances, when the fish swims below the single receiving channel transducer; and
**FIG. 13** is a flow chart illustrating a method for target size calculation, in accordance with an embodiment of the present disclosure.

Example apparatus are described herein. Other example embodiments or features may further be utilized, and other changes may be made, without departing from the scope of the appended claims. In the following detailed description, reference is made to the accompanying drawings, which form a part thereof.

The example embodiments described herein are not meant to be limiting. It will be readily understood that the aspects of the present disclosure, as generally described herein, and illustrated in the drawings, can be arranged, substituted, combined, separated, and designed in a wide variety of different configurations, all of which are explicitly contemplated herein.

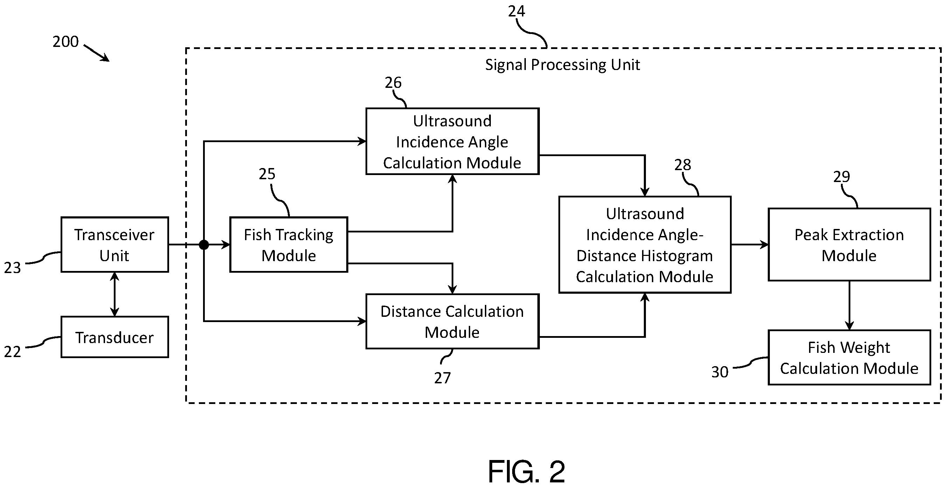

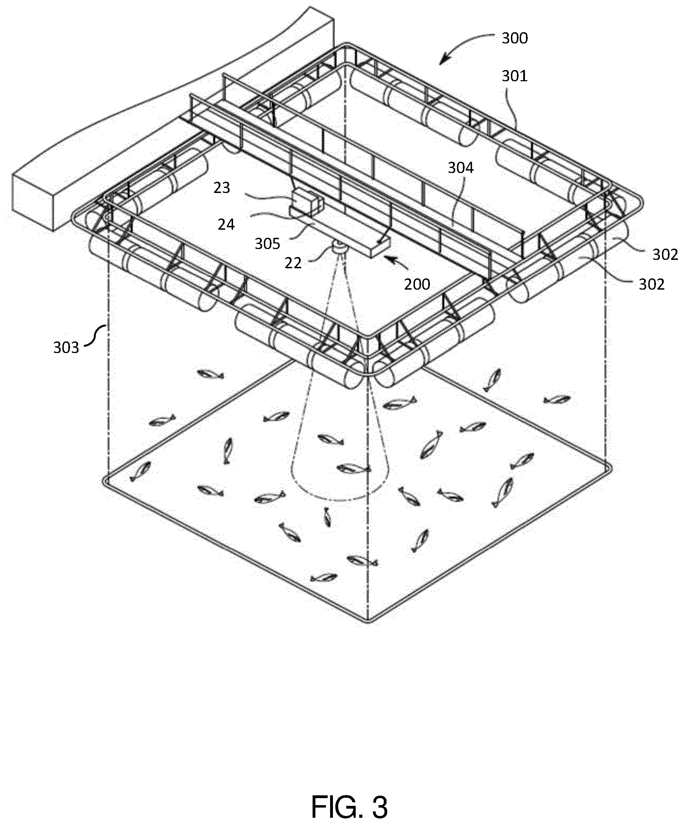

**FIG. 2** is a block diagram showing an overall configuration of a target size calculation device **200**, in accordance with a first embodiment of the present invention. **FIG. 3** is a perspective view showing how the target size calculation device **200** is used in a cage **300**.

The cage **300** is configured as a net-shaped cage, and includes a frame **301**, a float **302**, a net **303**, and a pier **304**. The frame **301** is formed so as to have a loop shape in a plan view. A plurality of floats **302** are attached to the frame **301**, whereby the frame **301** can be floated on the surface of the water. The frame **301** is connected to a weight on the bottom of the water by a mooring rope (not shown).

The upper end of the net **303** is fixed to the frame **301**. The net **303** is suspended so as to partition the water to form a closed space, and fish of known species are bred inside this space. The pier **304** for performing various operations related to aquaculture is fixed on the frame **301**.

A float **305** is floated in a substantially central portion inside the frame **301**, and the float **305** is connected to the pier **304** by a rope. The target size calculation device **200** of the present embodiment is arranged on the float **305**. As shown in **FIG. 2**, the target size calculation device **200** includes a transducer **22**, a transceiver unit **23**, and a signal processing unit **24**.

The transducer **22** can mutually convert an electric signal and ultrasonic vibration, and can detect underwater by using a transmitted wave which is an ultrasonic wave. In this embodiment, the transducer **22** is attached to the lower part of the float **305**. The transducer **22** is arranged to transmit downward and transmits ultrasonic waves from the upper side of the water toward the fish in the cage **300**.

In the present embodiment, the transducer **22** includes one transmitter and a receiver having a plurality of elements divided into four receiving channels.

In the present embodiment, the transducer **22** can acquire a position of an underwater target (such as fish) based on a difference in timing at which the four receiving channels receive a reflected wave reflected on the underwater target (that is, a phase difference of the received reflected wave). As a result, three-dimensional detection by a known split beam method is realized. The structure of the transducer **22** can be changed as appropriate.

The transceiver unit **23** is connected to the transducer **22** via an electric cable. The transceiver unit **23** can output an electric signal to the transducer **22** via the electric cable so that the transducer **22** transmits a transmitted wave. Further, the transceiver unit **23** can acquire via the electric cable an electric signal acquired by the transducer **22** based on the reflected wave. Then, the transceiver unit **23** can convert the electric signal acquired from the transducer **22** into a reception signal which is a digital signal.

The signal processing unit **24** includes a fish tracking module **25**, an ultrasound incidence angle calculation module **26**, a distance calculation module **27**, an ultrasound incidence angle-distance histogram calculation module **28**, a peak extraction module **29**, and a fish weight calculation module **30**. The signal processing unit **24** may be configured as a known computer, and may be communicatively coupled to the transceiver unit **23** through a communication cable to acquire a received signal from the transducer **22**. Specifically, the signal processing unit **24** may include a CPU, a ROM, a RAM, and the like. A program for realizing the target size calculation method of the present invention may be stored in the ROM or the like.

In general, the transducer **22** is configured to mutually convert an electric signal and ultrasonic vibration. At transmission time, the transducer **22** converts a high power transmission signal provided by the transceiver unit **23** into an ultrasonic wave and transmits the ultrasonic wave underwater. At reception time, the transducer **22** receives reflection wave reflected from underwater targets, converts the reflection wave into an electric signal and outputs the electrical signal back to the transceiver unit **23**. The transceiver unit **23** may be configured to amplify and filter the electric signal, A/D convert the electric signal (analog signal) into a reception signal which is a digital signal and store in a memory (not shown) of corresponding signal processing unit. The transducer **22** is configured to transmit a transmission wave toward underwater targets and generate an echo signal from a reflection of the transmission wave on the underwater targets. Examples of underwater targets include, but are not limited to fish, etc. The signal processing unit is configured to receive, store and process the echo signals to measure the length, breadth, height and/or weight of fish in water.

It may be noted that the calculation of fish weight is not limited to the fish raised in the fish cage **300**, for example, the target size calculation device **200** may be attached to a fishing boat and used as a device for estimating the fish weight for a school of fish swimming in the sea, when a fish species of the fish school can be estimated by the fisherman or otherwise measured by a conventional fish finder that can identify fish species.

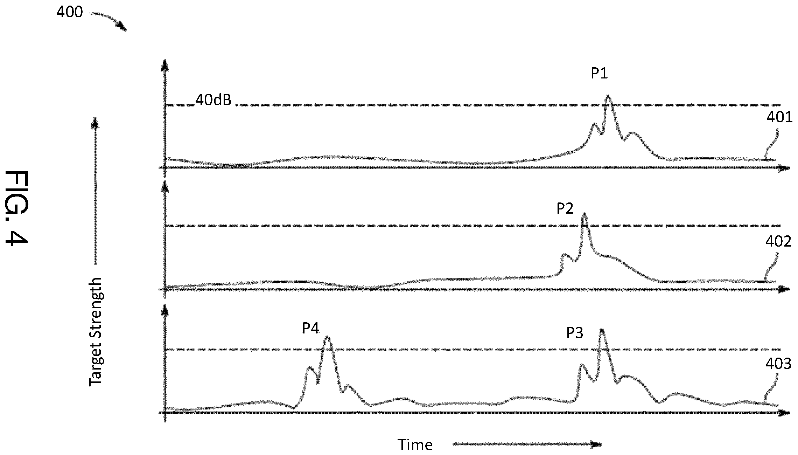

FIG. 4 illustrates an example of an envelope **400** of first, second and third echo signals **401**, **402** and **403** generated based on first, second and third transmission waves T1, T2 and T3 by the transducer **22** respectively. The first, second and third transmission waves T1, T2 and T3 are transmitted subsequently one after another, and corresponding echo signals **401**, **402** and **403** are stored in a memory (not shown).

In the envelope **400**, the horizontal axis is time and the vertical axis is Target Strength (TS) which indicates the strength of the received echo signal. The target strength is a parameter indicating the degree to which a part of the ultrasonic wave that is scattered by hitting the fish returns in the incident direction, and can be considered to be substantially the same as the echo intensity.

The fish tracking module **25** tracks in time a fish among the underwater targets from the first, second and third echo signals **401**, **402** and **403** resulting from different transmission waves. The fish tracking module **25** is configured to perform a swimbladder peak extraction in the echo signals **401**, **402** and **403**. The fish tracking module **25** determines whether or not the target strength value is equal to or higher than a predetermined detection threshold value. A peak of an echo signal that is above the given threshold is considered as an echo coming from the swimbladder of a fish, and is hereinafter also referred to as a swimbladder peak. In general, it is known that the ultrasonic waves transmitted from the transducer **22** are most strongly reflected on the swimbladder among each part of the fish. Therefore, as shown in **FIG. 4**, when the target strength (TS) shows the maximum peak, this peak can be considered to be derived from the reflected wave reflected by the swimbladder. A peak of an echo signal that is below the given threshold corresponds to an echo from other body parts of a fish such as back of fish. Herein, the threshold is -40dB, such that the first echo signal **401** includes a swimbladder peak P1, the second echo signal **402** includes a swimbladder peak P2, and the third echo signal **403** includes two swimbladder peaks P3 and P4.

For each swimbladder peak in a given echo signal, the fish tracking module **25** determines if there is a corresponding swimbladder peak for a previous transmission wave that occurred at about the same timing. If there is such a peak, the two swimbladder peak echoes are considered as actually coming from the same fish. In an example, peaks P1, P2 and P3 occur almost at the same timing, and therefore, can be considered coming from the same fish.

It would be apparent to one of ordinary skill in the art, that the fish tracking module **25** performs fish tracking by comparing peak positions on echo signals from two consecutive transmissions. However, there is no limitation on the number of consecutive transmissions, and more than two transmissions can be used for fish tracking. The fish tracking module **25** may employ sophisticated tracking algorithms for fish tracking, involving, for example, prediction by the use of Kalman filter.

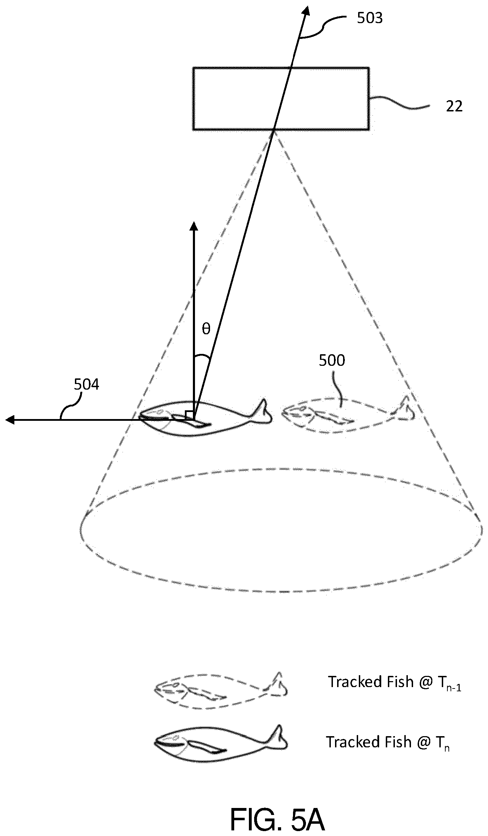

**FIG. 5A** illustrates a tracked fish **500** tracked at two positions using two consecutive transmissions $T_{n-1}$ and $T_n$ by the transducer 22. For each position of the tracked fish **500**, the ultrasound incidence angle calculation module **26** calculates an ultrasound incidence angle of the transmission wave on the tracked fish **500** based on the echo signals. Thus, the ultrasound incidence angle calculation module **26** calculates an angle at which the transmission wave penetrates into the tracked fish **500**. In the context of the present disclosure, the transducer **22** may include a plurality of transducer elements (which may also be referred to as channels) at reception. The ultrasound incidence angle calculation module **26** may calculate a direction of arrival (DOA) of the echoes from the fish **500** using the conventional interferometry principle, *i.e.,* by calculating the phase difference between the echo signals received by the plurality of transducer elements of the transducer **22**.

In the present embodiment, the transducer **22** may include multiple receiving channels and is capable of obtaining the arrival direction of the reflected wave by interferometry principle (also conventionally known as split beam method). Each receiving channel has a different directivity, and receives the reflected wave reflected from the target in the water. Then, the transducer **22** converts the data related to the received reflected wave into an electric signal. According to the split beam method, an angle between the arrival direction of the reflected wave and the direction of the central axis of the transmitted wave is calculated based on the difference of phase of the electrical signals received by the receiving channels.

The ultrasound incidence angle calculation module **26** calculates a 3D position of the tracked fish **500** at each transmission. Particularly, for each position, the ultrasound incidence angle calculation module **26** calculates a distance between the fish **500** and the transducer **22** based on time difference between transmission and reception of the echoes of the fish **500**. Then, the ultrasound incidence angle calculation module **26** calculates the 3D position of the fish **500** relative to the transducer **22** based on corresponding DOA of the fish echo and the distance between the fish **500** and the transducer **22**. The position of the fish **500** relative to the transducer **22** constitutes a fish-transducer vector **503**, which may vary from one transmission to another.

The ultrasound incidence angle calculation module **26** further calculates a swimming direction vector **504** of the fish **500** based on change in position of the fish **500** from a previous transmission $T_{n-1}$ to a current transmission $T_n$. The ultrasound incidence angle calculation module **26** calculates the ultrasound incidence angle by calculating an angle made between a first line (*i.e.,* the vector **504**) that connects two different positions of the tracked fish **500** in time and a second line (*i.e.,* the vector **503**) that connects one of the two different positions and a position of the transducer **22**. The ultrasound incidence angle calculation module **26** calculates, for each position of the tracked fish, the ultrasound incidence angle of the transmission wave on the tracked fish **500** based on the following equation:

Ultrasound incidence angle = 90° - angle (swimming direction vector **504**, fish-transducer vector **503**) ....          (1)

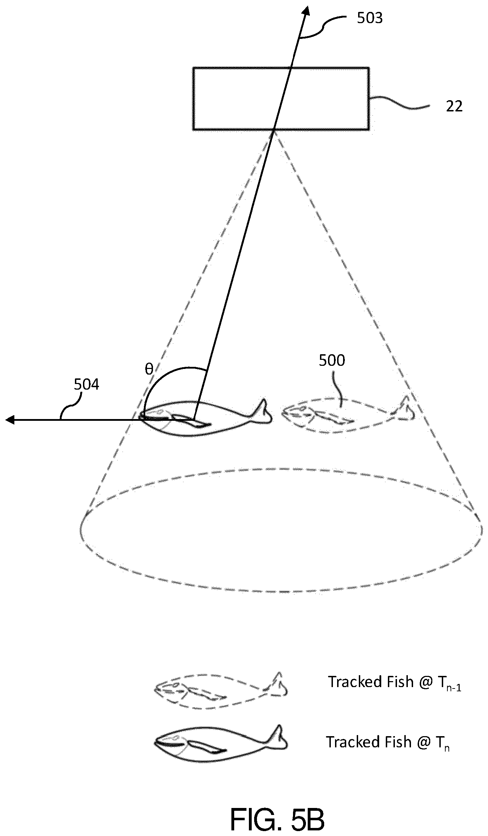

The ultrasound incidence angle is a complementary angle of an angle between the swimming direction vector **504** and the fish-transducer vector **503**. In an example, the ultrasound incidence angle is zero for a fish that is vertically below the transducer **22**, and swims horizontally. It would be apparent to one of ordinary skill in the art, that there is no limitation to the complementary angle explained above for the definition of the ultrasound incidence angle and that, for example, the angle between the swimming direction vector **504** and the fish-transducer vector **503**, as shown in **FIG. 5B**, could also be used as a definition of the ultrasound incidence angle. Thus, in another embodiment of the present disclosure, the ultrasound incidence angle may be calculated based on the following equation:

Ultrasound incidence angle = angle (swimming direction vector **504**, fish-transducer vector **503**) .......          (2)

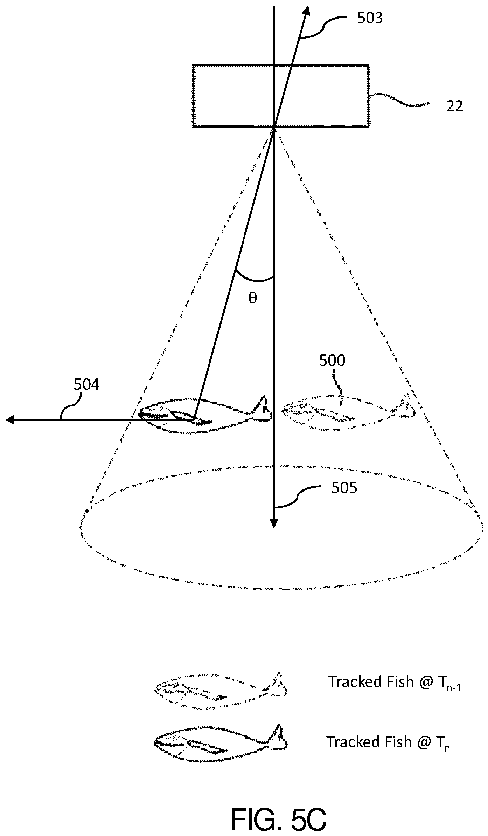

In yet another embodiment of the present disclosure, the ultrasound incidence angle calculation module **26** calculates the ultrasound incidence angle by calculating an angle made between the second line (*i.e.,* the fish-transducer vector **503**) that connects the position of the tracked fish **500** and the transducer **22** and a third line (*i.e.,* a vertical direction vector **505**) that is vertical line from the transducer **22** toward bottom of the water, as shown in **FIG. 5C**. Thus, the ultrasound incidence angle may be defined as an angle between the fish-transducer vector **503** and the vertical direction vector **505**. In this embodiment, the ultrasound incidence angle calculation module **26** calculates, for each position of the tracked fish **500**, the ultrasound incidence angle of the transmission wave on the tracked fish **500** based on the following equation:

Ultrasound incidence angle = angle (fish-transducer vector **503**, vertical direction vector **505**) ................ (3).

In the above embodiment, it is assumed that the transducer **22** is transmitting vertically downward from above the fish **500**. However, the transducer **22** may also be arranged to transmit vertically upward from below the fish **500**.

Although in the embodiment mentioned above, the transducer **22** includes a plurality of receiving channels and the ultrasound incidence angle is calculated based on a calculation of a position of the fish within a beam of the transducer **22** using the conventional split beam method, the scope of the present disclosure is not limited to it. In an alternate embodiment of the present disclosure, the transducer **22** includes a single receiving channel and the ultrasound incidence angle may be calculated using a suitable method. The calculation of the ultrasound incidence angle when the transducer **22** includes the single receiving channel has been explained later.

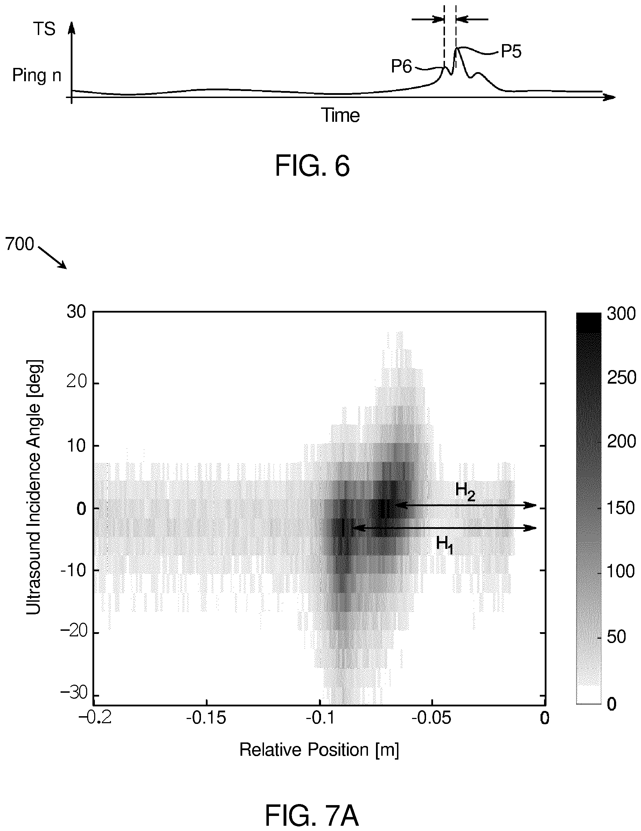

**FIG. 6** illustrates calculation of a swimbladder-back distance for the tracked fish **500** for a given transmission. The swimbladder-back distance is the distance between the swimbladder and back of the fish **500**, and is calculated based on time difference between the swimbladder peak P5 and peak P6 of the back of the fish **500**.

The distance calculation module **27** of **FIG. 2** calculates for each position of the tracked fish, a time difference between the peak P5 in the echo signal corresponding to the swimbladder and the peak P6 corresponding to another body part of the tracked fish. The distance calculation module **27** then converts the time difference into the swimbladder-back distance. It would be apparent to one of ordinary skill in the art, that another body part is not limited to back, and may include fish belly as another outer surface of the fish, or backbone of the fish as an inner part of the fish, etc.

In order to detect the echo reflected from the back of the fish **500**, the following processing may be performed. When the transducer **22** transmits the ultrasonic waves downward from above the fish **500**, the echo with a maximum echo intensity, *i.e.,* a target strength (TS), is considered as an echo reflected from the swimbladder of the fish **500** and a peak in the echo signal appearing immediately before that of the swimbladder peak is considered as an echo from the back of the fish **500**. In order to make sure with a high reliability that the peak before the swimbladder peak is actually the peak corresponding to the back of the fish **500**, a processing circuit (not shown) of the distance calculation module **27** may check the following condition. The processing checks within a given time interval of a given duration, extending before the peak corresponding to the back of the fish and starting from the peak corresponding to the back of the fish, whether another peak of at least a given echo intensity exists. If no such other peak exists within that given time interval, the peak appearing immediately before the swimbladder peak is considered as corresponding to the back of the fish with a high reliability.

In the above embodiments, the swimbladder-back of fish distance is calculated. However, there is no such limitation. The time difference between the swimbladder peak and any other peak within the echo signal of the same fish may be used instead.

**FIG. 7A** illustrates an ultrasound incidence angle vs swimbladder-back distance histogram **700** (*i.e.,* a 2D histogram), in accordance with an embodiment of the present disclosure. The ultrasound incidence angle-distance histogram calculation module **28** of **FIG. 2** calculates the ultrasound incidence angle vs swimbladder-back distance histogram **700** from the ultrasound incidence angle and swimbladder-back distance calculated for a large number of tracked fish. By repeatedly performing the calculation of the fish tracking module **25**, the ultrasound incidence angle calculation module **26**, and the distance calculation module **27** for a large number of transmit/receive cycles, a large number of tracked fish for which swimbladder-back distance and ultrasound incidence angle has been calculated can be obtained. This large number of swimbladder-back distance and ultrasound incidence angle data is used to generate the histogram **700**.

In order to generate the histogram **700**, the entire range of values of the swimbladder-back distance data and the entire range of values of the ultrasound incidence angle data are divided into a series of intervals and then the number of values (occurrence) that fall into each interval is counted. The histogram **700** represents this count number. The dark areas of the histogram **700** show combinations of (ultrasound incidence angle, swimbladder-back distance) that most often occur. In the histogram **700**, two dark areas can be seen, which illustrates ultrasound incidence angles in which swimbladder-back distance has been most often measured. As described above, the transducer **22** is installed so as to send the transmitted wave downward from near the water surface. Therefore, a negative value on the horizontal axis means the back side of the fish from the position of the swimbladder (reference position), and a positive value means the ventral side of the fish from the position of the swimbladder.

The peak extraction module **29** of **FIG. 2** extracts first and second peaks from the histogram **700** corresponding to the two dark areas in **FIG. 7A**, and calculates a first peak distance $H_1$ of the first peak, and a second peak distance $H_2$ of the second peak on the histogram **700**. The first and second peak distances $H_1$ and $H_2$ are illustrated with reference to **FIG. 7A**.

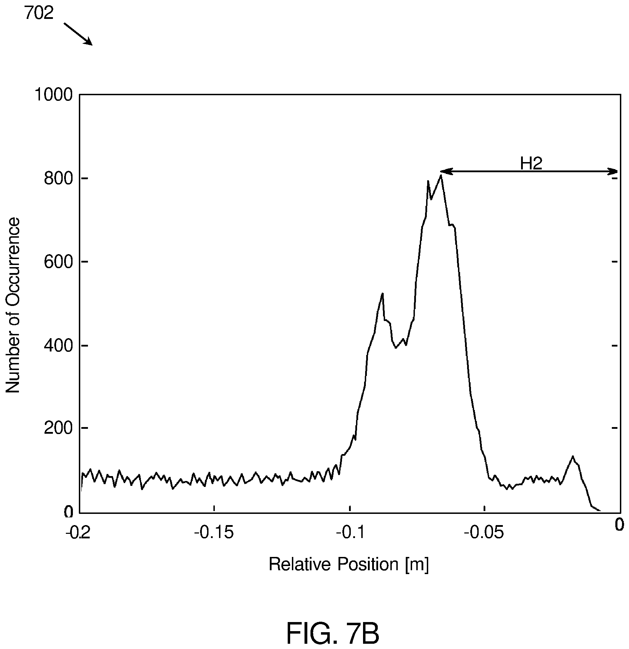

One method to extract the second peak from the histogram **700** is explained below. In order to extract the second peak, only the data in the histogram **700** having an ultrasound incidence angle between [0, 10deg] is extracted from the 2-D histogram **700** and rearranged into a 1-D histogram **702** illustrated in **FIG. 7B**, which illustrates a histogram (number of occurrence) of the swimbladder-back distance (*i.e.,* horizontal axis in **FIG. 7B**) for ultrasound incidence angles between [0, 10deg]. The horizontal axis of the 1-D histogram **702** is the same as the horizontal axis of the 2-D histogram **700**. The vertical axis of the 1-D histogram **702** represents the number of occurrences. A number of occurrences on the 1-D histogram **702** is equal to the sum of numbers of occurrence in the ultrasound incidence angle range [0, 10deg] of the 2-D histogram **700**. This 1-D histogram **702** which is focusing on ultrasound incidence angles only between [0, 10deg] more clearly emphasizes the second peak, *i.e.,* the peak on the right in both **FIGs. 7A** and **7B**.

In order to extract the first peak, only the data in the histogram **700** having an ultrasound incidence angle between [-20deg, 0] is extracted from the 2-D histogram **700** and rearranged into a 1-D histogram (not illustrated) using the same principle as explained above, in order to emphasize the first peak, *i.e.,* the peak on the left in **FIG. 7A.**

In order to remove noise in the 1-D histogram **702**, the peak extraction module **29** may apply a moving average on the 1-D histogram **702**.

The peak extraction module **29** then calculates the first peak distance $H_1$ of the first peak and the second peak distance $H_2$ of the second peak using respectively the 1-D histogram **702** that emphasizes the first peak and the 1-D histogram **702** that emphasizes the second peak. **FIG. 7B** illustrates the second peak distance $H_2$, which is the distance along the horizontal axis between a position of the second peak *(i.e.,* a position of the maximum of the second peak) on the horizontal axis and the reference position (swimbladder position) on the horizontal axis.

The above described method to extract the first and second peaks from the histogram **700** and calculate the first peak distance $H_1$ and the second peak distance $H_2$ performs a conversion of the 2-D histogram **700** into the 1-D histogram **702**. It would be apparent to one of ordinary skill in the art, that there is no limitation to this method as there are several other conventional methods to extract a peak and calculate a distance between that peak and a reference position. The [0, 10deg] range and [-20deg, 0] range used for the peak extraction are also not a limitation and may change depending on fish species in the cage **300**, maturity of the fish, etc.

The fish weight calculation module **30** of **FIG. 2** calculates a mean weight of the plurality of tracked fish from the first peak distance and the second peak distance. More specifically, the mean fish weight $W_m$ can be calculated as follows:

$$Wm = \alpha H_2^{\beta} H_1^{\gamma} \qquad \ldots\ldots\ldots\ldots\ldots(4)$$

where $\alpha$, $\beta$, $\gamma$ are predetermined values based on experiment data for a given fish species.

Although the ultrasound incidence angle-distance histogram **700** of **FIG. 7A** includes 2 peaks, it would be apparent to one of ordinary skill in the art, that there is no limitation on the number of peaks. Based on the fish species, the ultrasound incidence angle-distance histogram calculation module **28** may calculate a histogram that has more than two peaks, and calculate mean fish weight $W_m$ using the following equation:

$$Wm = \alpha_0 \prod_1^N H_n^{\alpha_n}\ldots\ldots\ldots\ldots\ldots\ldots\ldots\ldots\ldots\ldots\ldots(5)$$

where N is the number of peaks in the histogram, $H_n$ is the distance of each peak n on the histogram to the swimbladder, and $\alpha_n$ are predetermined values based on experiment for a given fish species.

Although not shown, the signal processing unit **24** may generate display data for displaying data relating to the size and weight of the fish. Then, the signal processing device **24** may output the display data to an appropriate display device connected to the signal processing unit **24**.

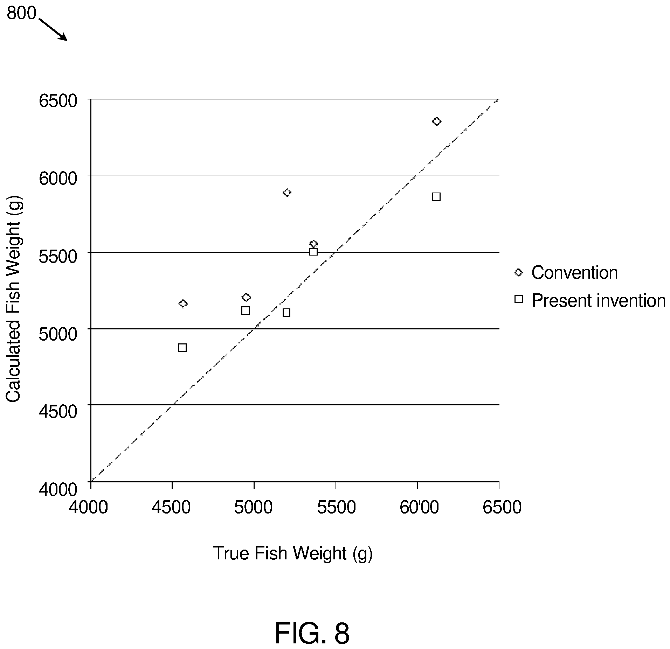

**FIG. 8** illustrates a graph **800** that shows result of five experiments performed with fish type yellowtail in which mean fish weight has been calculated using the conventional fish weight calculation method (as described in WO2020/090287) and the target size calculation device **200**.

In the graph **800**, the horizontal axis represents the true fish weight (mean weight of fish in the cage **300** measured with a scale) and the vertical axis represents the calculated mean fish weight with the conventional method and the method of the present disclosure. The conventional method yields on average a 7.8% error whereas the method of the present invention yields on average a 3.8% error.

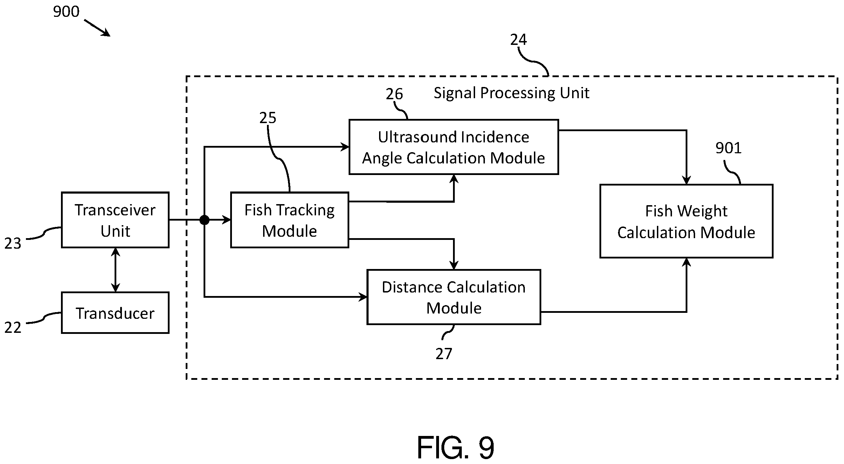

**FIG. 9** is a block diagram showing an overall configuration of a target size calculation device **900**, in accordance with a second embodiment of the present invention.

The target size calculation device **900** of the second embodiment differs from the target size calculation device **200** of the first embodiment in the configuration of a fish weight calculation module **901**. Also, the target size calculation device **900** does not include the ultrasound incidence angle-distance histogram calculation module and the peak extraction module of the first embodiment.

The fish weight calculation module **901** calculates fish weight for each tracked fish. Once a fish is tracked on a plurality of transmissions, the ultrasound incidence angle of the transmission wave on the tracked fish is calculated on each transmission by the ultrasound incidence angle calculation module **26**.

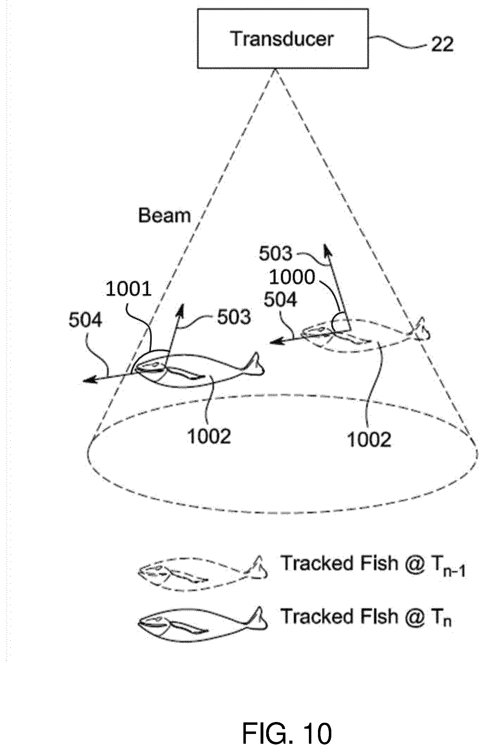

**FIG. 10** illustrates first and second ultrasound incidence angles **1000** and **1001** of a transmission wave on a tracked fish **1002** tracked on first and second transmissions $T_{n-1}$ and $T_n$ by the transducer **22** respectively. In **FIG. 10**, the first and second ultrasound incidence angles **1000** and **1001** are illustrated as defined by equation (2) and **FIG. 5B**, but there is no limitation and could also be illustrated as defined by equation (1) and **FIG. 5A** or as defined by equation (3) and **FIG. 5C**. The fish weight calculation module **901** compares each of the first and second ultrasound incidence angles with a given threshold angle, and calculates a weight of the tracked fish **1002** when the first ultrasound incidence angle and second ultrasound incidence angle are such that the given threshold angle is in-between the first and second ultrasound incidence angles. In an example, when the second ultrasound incidence angle is found to be greater than the given threshold angle, and the first ultrasound incidence angle is found to be less than the given threshold angle, the fish weight calculation module **901** extracts swimbladder-back distances in the first and second ultrasound incidence angles from the distance calculation module **27**. The fish weight calculation module **901** then calculates an individual fish weight $W_i$ of the tracked fish based on the following equation:

$$Wi = \alpha H_2^{\beta} H_1^{\gamma} \quad \ldots\ldots\ldots\ldots\ldots(6)$$

where, $H_1$ corresponds to the swimbladder-back distance in the first swimming direction and $H_2$ corresponds to the swimbladder-back distance in the second swimming direction, and $\alpha$, $\beta$, $\gamma$ are predetermined values based on experiment data for a given fish species.

When the first ultrasound incidence angle and second ultrasound incidence angle are not such that the given threshold angle is in-between the first and second ultrasound incidence angles, *i.e.,* when the first and second ultrasound incidence angles are both above the given threshold, or when the first and second ultrasound incidence angles are both below the given threshold, the fish weight calculation module **901** extracts swimbladder-back distance in one of the first and second ultrasound incidence angles from the distance calculation module 27. As an alternative, the fish weight calculation module **901** may extract swimbladder-back distances in both of the first and second ultrasound incidence angles and calculate an average of both swimbladder-back distances. The fish weight calculation module **901** then calculates the individual fish weight $W_i$ of the tracked fish based on the extracted swimbladder-back distance or the averaged swimbladder-back distance. In an example, when the first ultrasound incidence angle and the second ultrasound incidence angle are both found to be greater than the given threshold angle (*i.e.,* if H2 is only available), the equation (6) may be modified as below:

$$W_i = \alpha' H_2^{\beta'} \ldots\ldots\ldots\ldots\ldots(7)$$

where $\alpha'$, $\beta'$ are predetermined values based on experiment data for a given fish species.

Although, the fish weight calculation module **901** is shown to calculate a weight of the tracked fish based on tracking of fish on two transmissions, it would be apparent to one of ordinary skill in the art, that the fish weight may be calculated based on tracking of fish on more than two transmissions. In an example, when three transmissions are used for fish weight calculation, and if one ultrasound incidence angle is less than the given threshold angle and other two ultrasound incidence angles are more than the given threshold angle, then the swimbladder-back distances in the other two ultrasound incidence angles may be averaged before using them in the equation (6).

Thus, the fish weight calculation module **901** calculates the fish weight for each tracked fish, and determines a distribution of fish weight within a fish cage upon calculating fish weight of a plurality of tracked fish. The fish weight calculation module 901 provides more information, *i.e.,* fish weight distribution about weight of fish within the cage than the fish weight calculation module **30** of the first embodiment that calculates a mean weight of fish within the cage **300**.

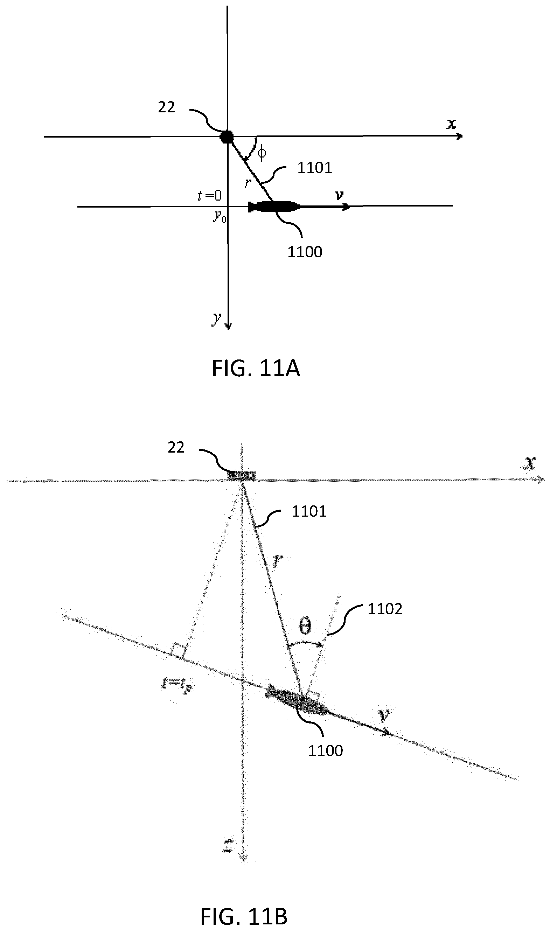

**FIG. 11A** illustrates a top view showing the calculation of the ultrasound incidence angle when the transducer **22** includes a single receiving channel, in accordance with an embodiment of the present disclosure. **FIG. 11B** illustrates a side view showing the calculation of the ultrasound incidence angle when the transducer **22** includes a single receiving channel, in accordance with an embodiment of the present disclosure.

As shown in **FIGs. 11A** and **11B,** a fish **1100** swims linearly below a single beam transducer **22** (*i.e.,* the transducer **22** that includes a single receiving channel) placed at a fixed position in the fish cage **300** and oriented vertically down. A 3D cartesian coordinate system is shown in the **FIGs. 11A** and **11B**, such that the transducer **22** is placed at a center of the 3D cartesian coordinate system. The fish **1100** is at a distance **r** from the transducer **22** and swims at a speed **v**.

At time t=0, the fish **1100** crosses the YZ plane. The distance **r** varies with time **t** according to the following equation (hyperbolic curve):

$$r^2 = v^2(t - t_p)^2 + r_p{}^2 \ldots\ldots\ldots\ldots.(8)$$

where $t_p$ corresponds to the time $t$ when the distance $r$ is minimum, and $r_p$ is the minimum distance between the fish **1100** and the transducer **22**.

As shown in **FIG. 11B**, the ultrasound incidence angle is an angle between a fish-transducer vector **1101** (*i.e.,* a first line that connects the fish **1100** and the transducer **22**) and a perpendicular vector **1102** (*i.e.,* a second line that is perpendicular to a direction in which the fish **1100** is swimming). The ultrasound incidence angle at time $t$ may be expressed using the following equation:

$$\theta = -\sin^{-1} \frac{v(t - t_p)}{r} \ldots \ldots \ldots \ldots \ldots \ldots (9)$$

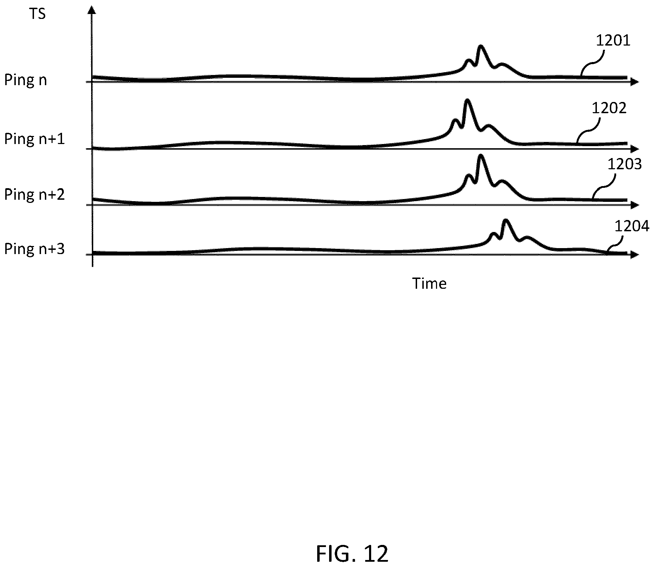

**FIG. 12** illustrates various echo signals, *i.e.,* echo signals **1201**, **1202**, **1203**, and **1204**, generated at different time instances *i.e.,* during pings **n**, **n+1**, **n+2**, and **n+3**, respectively, when the fish **1100** swims below the single receiving channel transducer **22**.

During each ping, a corresponding distance from the fish **1100** to the transducer **22** may be calculated by measuring a time taken by the ultrasonic wave to reflect from the swimbladder of the fish **1100**, by multiplying that time with the speed of sound in water, and by dividing the multiplication result by two. The distances r for each ping may be illustrated as below:

**Table I**

| Ping | Distance to fish |
|------|------------------|
| Ping n | $r_n$ |
| Ping n+1 | $r_{n+1}$ |
| Ping n+2 | $r_{n+2}$ |
| Ping n+3 | $T_{n+3}$ |

Referring back to **FIG.11B**, it is assumed that the distance between the fish **1100** and the transducer **22** changes with respect to time according to the below equation of a hyperbolic curve:

$$r^2 = at^2 + bt + c \ldots (10)$$

where r is the distance and t is time.

The parameters a, b, and c of the equation (10) are obtained using, for example, least squares method. Based on the equations (8) and (10), the speed of the fish v and the time $t_p$ are calculated as follows:

$$v = \sqrt{a}$$

$$t_p = -\frac{b}{2a}$$

Herein, the ultrasound incidence angle calculation module **26** calculates the ultrasound incidence angle using equation (9) at each ping by substituting the value of the speed **v** of the fish **1100**, and the time $t_p$. In the context of the present disclosure, when the transducer **22** generates the first and second echo signals **1201** and **1202** based on the first and second ultrasonic waves (*i.e.,* the first and second pings **n** and **n+1**), the first and second distances $r_n$ and $r_{n+1}$ are calculated as described above, respectively. In the embodiment, the second ultrasonic wave is transmitted after the first ultrasonic wave. Thus, at the second time instance, the ultrasound incidence angle calculation module **26** calculates a second incidence angle $\theta_{n+1}$ based on the first distance $r_n$ between the aquatic animal (*i.e.,* the fish **1100**) and the transducer **22** in the first echo signal **1201** and the second distance $r_{n+1}$ between the aquatic animal and the transducer **22** in the second echo signal **1202**. The ultrasound incidence angle calculation module **26** calculates the second incidence angle $\theta_{n+1}$, based on an assumption that the first and second distances $r_n$ and $r_{n+1}$ change with time based on a hyperbolic curve, using the equation (10).

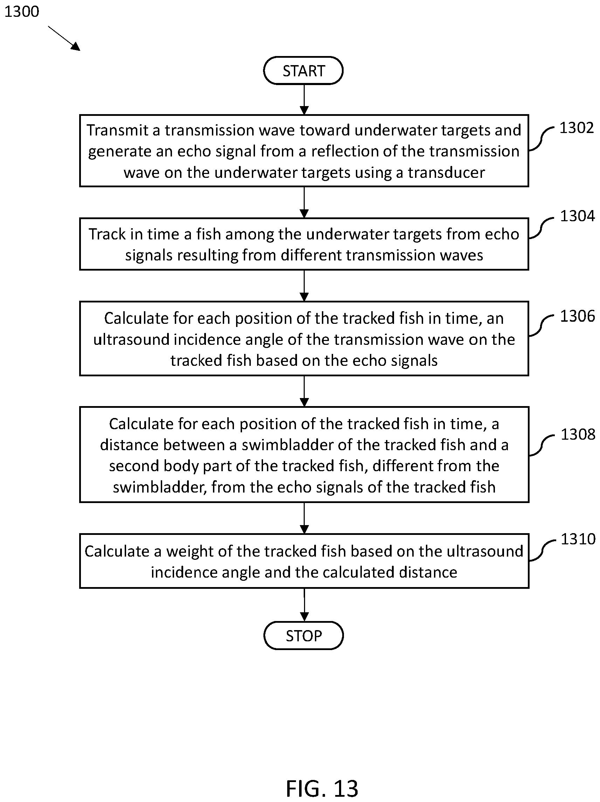

**FIG. 13** is a flow chart illustrating a method **1300** for target size calculation, in accordance with an embodiment of the present disclosure. Various steps of the method have been explained with reference to **FIGs. 2** and **9**.

At step **1302**, the transducer **22** transmits a transmission wave toward underwater targets and generate an echo signal from a reflection of the transmission wave on the underwater targets.

At step **1304**, the fish tracking module **25** tracks in time a fish among the underwater targets from echo signals resulting from different transmission waves.

At step **1306**, the ultrasound incidence angle calculation module **26** calculates, for each position of the tracked fish in time, an ultrasound incidence angle of the transmission wave on the tracked fish based on the echo signals.

At step **1308**, the distance calculation module **27** calculates, for each position of the tracked fish in time, a distance between a swimbladder of the tracked fish and a second body part of the tracked fish, different from the swimbladder, from the echo signals of the tracked fish.

At step **1310**, the fish weight calculation module **30** and **901** calculates a weight of the tracked fish based on the ultrasound incidence angle and the calculated distance.

It is to be understood that not necessarily all objectives or advantages may be achieved in accordance with any particular embodiment described herein. Thus, for example, those skilled in the art will appreciate that certain embodiments may be configured to operate in a manner that achieves or optimizes one advantage or group of advantages as taught herein without necessarily achieving other objects or advantages as may be taught or suggested herein.

All processes described herein may be embodied in, and fully automated via, software code modules executed by a computing system that includes one or more computers or processors. The software code modules may be stored in any type of non-transitory computer-readable medium or other computer storage device. Some or all methods may be embodied in specialized computer hardware.

Many other variations other than those described herein will be apparent from this disclosure. For example, depending on the embodiment, certain actions, events, or functions of any of the algorithms described herein may be performed in different sequences, and may be added, merged, or excluded altogether (e.g., not all described actions or events are required to execute the algorithm). Moreover, in certain embodiments, operations or events are performed in parallel, for example, through multithreading, interrupt handling, or through multiple processors or processor cores, or on other parallel architectures, rather than sequentially. In addition, different tasks or processes can be performed by different machines and/or computing systems that can work together.

The various exemplary logical blocks and modules described in connection with the embodiments disclosed herein can be implemented or executed by a machine such as a processor. The processor may be a microprocessor, but alternatively, the processor may be a controller, a microcontroller, or a state machine, or a combination thereof. The processor can include an electrical circuit configured to process computer executable instructions. In another embodiment, the processor includes an application specific integrated circuit (ASIC), a field programmable gate array (FPGA), or other programmable device that performs logical operations without processing computer executable instructions. The processor can also be implemented as a combination of computing devices, e.g., a combination of a digital signal processor (DSP) and a microprocessor, a plurality of microprocessors, one or more microprocessors in conjunction with a DSP core, or any other such configuration. Although described herein primarily with respect to digital technology, the processor may also include primarily analog components. For example, some or all of the signal processing algorithms described herein may be implemented by analog circuitry or mixed analog and digital circuitry. A computing environment may include any type of computer system, including, but not limited to, a computer system that is based on a microprocessor, mainframe computer, a digital signal processor, a portable computing device, a device controller, or a computing engine within the device.

Unless otherwise stated, conditional languages such as "can", "could", "will", "might", or "may" are understood within the context as used in general to convey that certain embodiments include, while other embodiments do not include, certain features, elements and/or steps. Thus, such conditional languages are not generally intended to imply that features, elements and/or steps are in any way required for one or more embodiments or that one or more embodiments necessarily include logic for deciding, with or without user input or prompting, whether these features, elements and/or steps are included or are to be performed in any particular embodiment.

Disjunctive languages, such as the phrase "at least one of X, Y, or Z", unless specifically stated otherwise, is understood with the context as used in general to present that an item, term, etc., may be either X, Y, or Z, or any combination thereof (e.g., X, Y, and/or Z). Thus, such a disjunctive language is not generally intended to, and should not, imply that certain embodiments require at least one of X, at least one of Y, or at least one of Z to each be present.

Any process descriptions, elements, or blocks in the flow diagrams described herein and/or shown in the accompanying drawings should be understood as potentially representing modules, segments, or parts of code, including one or more executable instructions for implementing a particular logical function or elements in the process. Alternate implementations are included within the scope of the embodiments described herein in which elements or functions may be deleted, executed out of order from that shown, or discussed, including substantially concurrently or in reverse order, depending on the functionality involved as would be understood by those skilled in the art.

Unless otherwise explicitly stated, articles such as "a" or "an" should generally be interpreted to include one or more described items. Accordingly, phrases such as "a device configured to" are intended to include one or more recited devices. Such one or more recited devices can also be collectively configured to carry out the stated recitations. For example, "a processor configured to carry out recitations A, B, and C" can include a first processor configured to carry out recitation A working in conjunction with a second processor configured to carry out recitations B and C. The same holds true for the use of definite articles used to introduce embodiment recitations. In addition, even if a specific number of an introduced embodiment recitation is explicitly recited, those skilled in the art will recognize that such recitation should typically be interpreted to mean at least the recited number (e.g., the bare recitation of "two recitations", without other modifiers, typically means at least two recitations, or two or more recitations).

It will be understood by those within the art that, in general, terms used herein, are generally intended as "open" terms (e.g., the term "including" should be interpreted as "including but not limited to", the term "having" should be interpreted as "having at least", the term "includes" should be interpreted as "includes but is not limited to", etc.).

For expository purposes, the term "horizontal" as used herein is defined as a plane parallel to the plane or surface of the floor of the area in which the system being described is used or the method being described is performed, regardless of its orientation. The term "floor" can be interchanged with the term "ground" or "water surface". The term "vertical" refers to a direction perpendicular to the horizontal as just defined. Terms such as "above", "below", "bottom", "top", "side", "higher", "lower", "upper", "over", and "under", are defined with respect to the horizontal plane.

As used herein, the terms "attached", "connected", "mated" and other such relational terms should be construed, unless otherwise noted, to include removable, moveable, fixed, adjustable, and/or releasable connections or attachments. The connections/attachments can include direct connections and/or connections having intermediate structure between the two components discussed.

Numbers preceded by a term such as "approximately", "about", and "substantially" as used herein include the recited numbers, and also represent an amount close to the stated amount that still performs a desired function or achieves a desired result. For example, the terms "approximately", "about", and "substantially" may refer to an amount that is within less than 10% of the stated amount. Features of embodiments disclosed herein preceded by a term such as "approximately", "about", and "substantially" as used herein represent the feature with some variability that still performs a desired function or achieves a desired result for that feature.

It should be emphasized that many variations and modifications may be made to the above-described embodiments as long as they fall under the scope of the following claims.

## Claims

1. A target size calculation device, comprising:

   a transducer (22) configured to transmit a transmission wave toward underwater targets and generate an echo signal from a reflection of the transmission wave on the underwater targets;
   a fish tracking module (25) configured to track in time a fish among the underwater targets from echo signals resulting from different transmission waves;
   an ultrasound incidence angle calculation module (26) configured to calculate, for each position of the tracked fish in time, an ultrasound incidence angle of the transmission wave on the tracked fish based on the echo signals;
   a distance calculation module (27) configured to calculate, for each position of the tracked fish in time, a distance between a swimbladder of the tracked fish and a second body part of the tracked fish, different from the swimbladder, from the echo signals of the tracked fish;
   a fish weight calculation module (30, 901) configured to calculate a weight of the tracked fish based on the ultrasound incidence angle and the calculated distance; and
   an ultrasound incidence angle-distance histogram calculation module (28) configured to calculate an ultrasound incidence angle vs. distance histogram from the ultrasound incidence angle and the distance calculated for a plurality of tracked fish.

2. The target size calculation device of claim 1, further comprising:
   a peak extraction module (29) configured to extract a first peak and a second peak from the histogram, and calculate a first peak distance of the first peak on the histogram and a second peak distance of the second peak on the histogram.

3. The target size calculation device of claim 2, wherein:
   the fish weight calculation module (30) is configured to calculate a mean weight of the plurality of tracked fish from the first peak distance and the second peak distance.

4. A target size calculation device, comprising:

a transducer (22) configured to transmit a transmission wave toward underwater targets and generate an echo signal from a reflection of the transmission wave on the underwater targets;

a fish tracking module (25) configured to track in time a fish among the underwater targets from echo signals resulting from different transmission waves;

an ultrasound incidence angle calculation module (26) configured to calculate, for each position of the tracked fish in time, an ultrasound incidence angle of the transmission wave on the tracked fish based on the echo signals;

a distance calculation module (27) configured to calculate, for each position of the tracked fish in time, a distance between a swimbladder of the tracked fish and a second body part of the tracked fish, different from the swimbladder, from the echo signals of the tracked fish; and

a fish weight calculation module (30, 901) configured to calculate a weight of the tracked fish based on the ultrasound incidence angle and the calculated distance;

wherein the fish weight calculation module (901) is configured to calculate a weight of the tracked fish when a first ultrasound incidence angle of the transmission wave on the tracked fish at a first timing and a second ultrasound incidence angle of the transmission wave on the tracked fish at a second timing different from the first timing are such that a given threshold angle is in-between the first and second ultrasound incidence angles; and wherein the fish weight calculation module (901) calculates the weight based on the distance of the tracked fish in the first ultrasound incidence angle and the distance of the tracked fish in the second ultrasound incidence angle.

5. The target size calculation device of any one of claims 1 to 4, wherein:

the ultrasound incidence angle calculation module (26) calculates the ultrasound incidence angle by calculating an angle made between a first line that connects two different positions of the tracked fish in time and a second line that connects one of the two different positions and a position of the transducer.

6. The target size calculation device of any one of claims 1 to 4, wherein:

the ultrasound incidence angle calculation module (26) calculates the ultrasound incidence angle by calculating an angle made between a second line that connects a position of the tracked fish and the transducer and a third line that is a vertical line from the transducer (22) toward bottom of the water.

7. The target size calculation device of any one of claims 1 to 4, wherein:

the ultrasound incidence angle calculation module (26) calculates the ultrasound incidence angle based on a first distance between the transducer and a first position of the tracked fish at a first time instance and a second distance between the transducer and a second position of the tracked fish at a second time instance different from the first time instance; wherein:

the ultrasound incidence angle calculation module (26) calculates the ultrasound incidence angle based on an assumption that the first and second distances change with time based on a hyperbolic curve.

8. The target size calculation device of any one of the previous claims, wherein:

the second body part is an outer surface of the tracked fish.

9. A target size calculation method, comprising:

transmitting a transmission wave toward underwater targets and generating an echo signal from a reflection of the transmission wave on the underwater targets using transducer;

tracking in time a fish among the underwater targets from echo signals resulting from different transmission waves;

calculating for each position of the tracked fish in time, an ultrasound incidence angle of the transmission wave on the tracked fish based on the echo signals;

calculating for each position of the tracked fish in time, a distance between a swimbladder of the tracked fish and a second body part of the tracked fish, different from the swimbladder, from the echo signals of the tracked fish;

calculating a weight of the tracked fish based on the ultrasound incidence angle and the calculated distance; and

calculating an ultrasound incidence angle vs. distance histogram from the ultrasound incidence angle and the distance calculated for a plurality of tracked fish.

10. A non-transitory computer readable medium having stored thereon computer-executable instructions which, when executed by the device of claim 1, cause the device to execute the method of claim 9.

11. A target size calculation method, comprising:

transmitting a transmission wave toward underwater targets and generating an echo signal from a reflection of the transmission wave on the underwater targets using transducer;

tracking in time a fish among the underwater targets from echo signals resulting from different transmission waves;

calculating for each position of the tracked fish in time, an ultrasound incidence angle of the transmission wave on the tracked fish based on the echo signals;

calculating for each position of the tracked fish in time, a distance between a swimbladder of the tracked fish and a second body part of the tracked fish, different from the swimbladder, from the echo signals of the tracked fish; and

calculating a weight of the tracked fish based on the ultrasound incidence angle and the calculated distance;

wherein the weight of the tracked fish is calculated when a first ultrasound incidence angle of the transmission wave on the tracked fish at a first timing and a second ultrasound incidence angle of the transmission wave on the tracked fish at a second timing different from the first timing are such that a given threshold angle is in-between the first and second ultrasound incidence angles, and wherein the weight is calculated based on the distance of the tracked fish in the first ultrasound incidence angle and the distance of the tracked fish in the second ultrasound incidence angle.

12. A non-transitory computer readable medium having stored thereon computer-executable instructions which, when executed by the device of claim 4, cause the device to execute the method of claim 11.

**Patentansprüche**

1. Zielgrößenberechnungsvorrichtung, die Folgendes umfasst:

einen Wandler (22), konfiguriert zum Übertragen einer Übertragungswelle zu Unterwasserzielen und zum Erzeugen eines Echosignals aus einer Reflexion der Übertragungswelle an den Unterwasserzielen;

ein Fischverfolgungsmodul (25), konfiguriert zum zeitlichen Verfolgen eines Fischs unter den Unterwasserzielen anhand von Echosignalen, die von verschiedenen Übertragungswellen stammen;

ein Ultraschalleinfallswinkel-Berechnungsmodul (26), konfiguriert zum Berechnen, für jede Position des zeitlich verfolgten Fischs, eines Ultraschalleinfallswinkels der Übertragungswelle auf den verfolgten Fisch auf der Basis der Echosignale;

ein Distanzberechnungsmodul (27), konfiguriert zum Berechnen, für jede Position des zeitlich verfolgten Fischs, einer Distanz zwischen einer Schwimmblase des verfolgten Fischs und einem zweiten Körperteil des verfolgten Fischs, der sich von der Schwimmblase unterscheidet, aus den Echosignalen des verfolgten Fischs;

ein Fischgewichtberechnungsmodul (30, 901), konfiguriert zum Berechnen eines Gewichts des verfolgten Fischs auf der Basis des Ultraschalleinfallswinkels und der berechneten Distanz; und

ein Ultraschalleinfallswinkel-Distanz-Histogramm-Berechnungsmodul (28), konfiguriert zum Berechnen eines Ultraschalleinfallswinkel-Distanz-Histogramms aus dem Ultraschalleinfallswinkel und der für mehrere verfolgte Fische berechneten Distanz.

2. Zielgrößenberechnungsvorrichtung nach Anspruch 1, die ferner Folgendes umfasst:
ein Peakextraktionsmodul (29), konfiguriert zum Extrahieren eines ersten Peaks und eines zweiten Peaks aus dem Histogramm und zum Berechnen einer ersten Peakdistanz des ersten Peaks auf dem Histogramm und einer zweiten Peakdistanz des zweiten Peaks auf dem Histogramm.

3. Zielgrößenberechnungsvorrichtung nach Anspruch 2, wobei:
das Fischgewichtberechnungsmodul (30) zum Berechnen eines Durchschnittsgewichts der mehreren verfolgten Fische aus der ersten Peakdistanz und der zweiten Peakdistanz konfiguriert ist.

4. Zielgrößenberechnungsvorrichtung, die Folgendes umfasst:

einen Wandler (22), konfiguriert zum Übertragen einer Übertragungswelle zu Unterwasserzielen und zum Erzeugen eines Echosignals aus einer Reflexion der Übertragungswelle an den Unterwasserzielen;

ein Fischverfolgungsmodul (25), konfiguriert zum zeitlichen Verfolgen eines Fischs unter den Unterwasserzielen anhand von Echosignalen, die von verschiedenen Übertragungswellen stammen;

ein Ultraschalleinfallswinkel-Berechnungsmodul (26), konfiguriert zum Berechnen, für jede Position des zeitlich verfolgten Fischs, eines Ultraschalleinfallswinkels der Übertragungswelle auf den verfolgten Fisch auf der Basis der Echosignale;

ein Distanzberechnungsmodul (27), konfiguriert zum Berechnen, für jede Position des zeitlich verfolgten Fischs, einer Distanz zwischen einer Schwimmblase des verfolgten Fischs und einem zweiten Körperteil des verfolgten Fischs, der sich von der Schwimmblase unterscheidet, aus den Echosignalen des verfolgten Fischs;

ein Fischgewichtberechnungsmodul (30, 901), konfiguriert zum Berechnen eines Gewichts des verfolgten Fischs auf der Basis des Ultraschalleinfallswinkels und der berechneten Distanz;

wobei das Fischgewichtberechnungsmodul (901) zum Berechnen eines Gewichts des verfolgten Fischs konfiguriert ist, wenn ein erster Ultraschalleinfallswinkel der Übertragungswelle auf den verfolgten Fisch zu einem ersten Zeitpunkt und ein zweiter Ultraschalleinfallswinkel der Übertragungswelle auf den verfolgten Fisch zu einem zweiten Zeitpunkt, der sich vom ersten Zeitpunkt unterscheidet, so sind, dass ein gegebener Schwellenwinkel zwischen dem ersten und dem zweiten Ultraschalleinfallswinkel liegt; und wobei das Fischgewichtberechnungsmodul (901) das Gewicht auf der Basis der Distanz des verfolgten Fischs im ersten Ultraschalleinfallswinkel und der Distanz des verfolgten Fischs im zweiten Ultraschalleinfallswinkel berechnet.

5. Zielgrößenberechnungsvorrichtung nach einem der Ansprüche 1 bis 4, wobei:
das Ultraschalleinfallswinkel-Berechnungsmodul (26) den Ultraschalleinfallswinkel durch Berechnen eines Winkels berechnet, der zwischen einer zwei verschiedene Positionen des zeitlich verfolgten Fischs verbindenden ersten Linie und einer eine der zwei verschiedenen Positionen und eine Position des Wandlers verbindenden zweiten Linie gebildet wird.

6. Zielgrößenberechnungsvorrichtung nach einem der Ansprüche 1 bis 4, wobei:
das Ultraschalleinfallswinkel-Berechnungsmodul (26) den Ultraschalleinfallswinkel durch Berechnen eines Winkels berechnet, der zwischen einer eine Position des verfolgten Fischs und den Wandler verbindenden zweiten Linie und einer dritten Linie gebildet wird, die eine vertikale Linie vom Wandler (22) zum Grund des Wassers ist.

7. Zielgrößenberechnungsvorrichtung nach einem der Ansprüche 1 bis 4, wobei:
das Ultraschalleinfallswinkel-Berechnungsmodul (26) den Ultraschalleinfallswinkel auf der Basis einer ersten Distanz zwischen dem Wandler und einer ersten Position des verfolgten Fischs zu einem ersten Zeitpunkt und einer zweiten Distanz zwischen dem Wandler und einer zweiten Position des verfolgten Fischs zu einem zweiten Zeitpunkt, der sich vom ersten Zeitpunkt unterscheidet, berechnet; wobei:
das Ultraschalleinfallswinkel-Berechnungsmodul (26) den Ultraschalleinfallswinkel auf der Basis einer Annahme berechnet, dass sich die erste und zweite Distanz zeitlich auf der Basis einer Hyperbelkurve ändern.

8. Zielgrößenberechnungsvorrichtung nach einem der vorherigen Ansprüche, wobei:
der zweite Körperteil eine Außenfläche des verfolgten Fischs ist.

9. Zielgrößenberechnungsverfahren, das Folgendes beinhaltet:

Übertragen einer Übertragungswelle zu Unterwasserzielen und Erzeugen eines Echosignals aus einer Reflexion der Übertragungswelle an den Unterwasserzielen mittels eines Wandlers;

zeitliches Verfolgen eines Fischs unter den Unterwasserzielen anhand von Echosignalen, die von verschiedenen Übertragungswellen stammen;

Berechnen, für jede Position des zeitlich verfolgten Fischs, eines Ultraschalleinfallswinkels der Übertragungswelle auf den verfolgten Fisch auf der Basis der Echosignale;

Berechnen, für jede Position des zeitlich verfolgten Fischs, einer Distanz zwischen einer Schwimmblase des verfolgten Fischs und einem zweiten Körperteil des verfolgten Fischs, der sich von der Schwimmblase unterscheidet, aus den Echosignalen des verfolgten Fischs;

Berechnen eines Gewichts des verfolgten Fischs auf der Basis des Ultraschalleinfallswinkels und der berechneten Distanz; und

Berechnen eines Ultraschalleinfallswinkel-Distanz-Histogramms aus dem Ultraschalleinfallswinkel und der für mehrere verfolgte Fische berechneten Distanz.

10. Nicht-transitorisches computerlesbares Medium, auf dem computerausführbare Befehle gespeichert sind, die bei Ausführung durch die Vorrichtung nach Anspruch 1 die Vorrichtung zum Durchführen des Verfahrens nach Anspruch 9 veranlassen.

11. Zielgrößenberechnungsverfahren, das Folgendes beinhaltet:

Übertragen einer Übertragungswelle zu Unterwasserzielen und Erzeugen eines Echosignals aus einer Reflexion

EP 4 067 933 B1

der Übertragungswelle an den Unterwasserzielen mittels eines Wandlers;
zeitliches Verfolgen eines Fischs unter den Unterwasserzielen anhand von Echosignalen, die von verschiedenen Übertragungswellen stammen;
Berechnen, für jede Position des zeitlich verfolgten Fischs, eines Ultraschalleinfallswinkels der Übertragungswelle auf den verfolgten Fisch auf der Basis der Echosignale;
Berechnen, für jede Position des zeitlich verfolgten Fischs, einer Distanz zwischen einer Schwimmblase des verfolgten Fischs und einem zweiten Körperteil des verfolgten Fischs, der sich von der Schwimmblase unterscheidet, aus den Echosignalen des verfolgten Fischs; und
Berechnen eines Gewichts des verfolgten Fischs auf der Basis des Ultraschalleinfallswinkels und der berechneten Distanz;
wobei das Gewicht des verfolgten Fischs berechnet wird, wenn ein erster Ultraschalleinfallswinkel der Übertragungswelle auf den verfolgten Fisch zu einem ersten Zeitpunkt und ein zweiter Ultraschalleinfallswinkel der Übertragungswelle auf den verfolgten Fisch zu einem zweiten Zeitpunkt, der sich vom ersten Zeitpunkt unterscheidet, so sind, dass ein gegebener Schwellenwinkel zwischen dem ersten und dem zweiten Ultraschalleinfallswinkel liegt; und wobei das Gewicht auf der Basis der Distanz des verfolgten Fischs im ersten Ultraschalleinfallswinkel und der Distanz des verfolgten Fischs im zweiten Ultraschalleinfallswinkel berechnet wird.

12. Nicht-transitorisches computerlesbares Medium, auf dem computerausführbare Befehle gespeichert sind, die bei Ausführung durch die Vorrichtung nach Anspruch 4 die Vorrichtung zum Durchführen des Verfahrens nach Anspruch 11 veranlassen.

**Revendications**

1. Dispositif de calcul de taille de cible, comprenant :

un transducteur (22), configuré pour émettre une onde d'émission vers des cibles sous-marines et pour générer un signal d'écho à partir d'une réflexion de l'onde d'émission sur les cibles sous-marines ;
un module de suivi de poisson (25), configuré pour suivre dans le temps un poisson parmi les cibles sous-marines à partir de signaux d'écho résultant de différentes ondes d'émission ;
un module de calcul d'angle d'incidence d'ultrasons (26), configuré pour calculer, pour chaque position du poisson suivi dans le temps, un angle d'incidence d'ultrasons de l'onde d'émission sur le poisson suivi, sur la base des signaux d'écho ;
un module de calcul de distance (27), configuré pour calculer, pour chaque position du poisson suivi dans le temps, une distance entre une vessie natatoire du poisson suivi et une seconde partie corporelle du poisson suivi, différente de la vessie natatoire, à partir des signaux d'écho du poisson suivi ;
un module de calcul de poids de poisson (30, 901), configuré pour calculer un poids du poisson suivi sur la base de l'angle d'incidence d'ultrasons et de la distance calculée ; et
un module de calcul d'histogramme angle d'incidence d'ultrasons-distance (28), configuré pour calculer un histogramme de l'angle d'incidence d'ultrasons en fonction de la distance à partir de l'angle d'incidence d'ultrasons et de la distance, calculés pour une pluralité de poissons suivis.

2. Dispositif de calcul de taille de cible selon la revendication 1, comprenant en outre :
un module d'extraction de crête (29), configuré pour extraire une première crête et une seconde crête de l'histogramme et pour calculer une première distance de crête de la première crête sur l'histogramme et une seconde distance de crête de la seconde crête sur l'histogramme.

3. Dispositif de calcul de taille de cible selon la revendication 2, dans lequel :
le module de calcul de poids de poisson (30) est configuré pour calculer un poids moyen de la pluralité de poissons suivis à partir de la première distance de crête et de la seconde distance de crête.

4. Dispositif de calcul de taille de cible, comprenant :

un transducteur (22), configuré pour émettre une onde d'émission vers des cibles sous-marines et pour générer un signal d'écho à partir d'une réflexion de l'onde d'émission sur les cibles sous-marines ;
un module de suivi de poisson (25), configuré pour suivre dans le temps un poisson parmi les cibles sous-marines à partir de signaux d'écho résultant de différentes ondes d'émission ;
un module de calcul d'angle d'incidence d'ultrasons (26), configuré pour calculer, pour chaque position du

16

poisson suivi dans le temps, un angle d'incidence d'ultrasons de l'onde d'émission sur le poisson suivi, sur la base des signaux d'écho ;

un module de calcul de distance (27), configuré pour calculer, pour chaque position du poisson suivi dans le temps, une distance entre une vessie natatoire du poisson suivi et une seconde partie corporelle du poisson suivi, différente de la vessie natatoire, à partir des signaux d'écho du poisson suivi ; et

un module de calcul de poids de poisson (30, 901), configuré pour calculer un poids du poisson suivi sur la base de l'angle d'incidence d'ultrasons et de la distance calculée ;

le module de calcul de poids de poisson (901) étant configuré pour calculer un poids du poisson suivi lorsqu'un premier angle d'incidence d'ultrasons de l'onde d'émission sur le poisson suivi à un premier moment et un second angle d'incidence d'ultrasons de l'onde d'émission sur le poisson suivi à un second moment différent du premier moment sont tels qu'un angle seuil donné se situe entre les premier et second angles d'incidence d'ultrasons ; et le module de calcul de poids de poisson (901) calculant le poids sur la base de la distance du poisson suivi dans le premier angle d'incidence d'ultrasons et de la distance du poisson suivi dans le second angle d'incidence d'ultrasons.

5. Dispositif de calcul de taille de cible selon l'une quelconque des revendications 1 à 4, dans lequel :
le module de calcul d'angle d'incidence d'ultrasons (26) calcule l'angle d'incidence d'ultrasons en calculant un angle formé entre une première ligne qui relie deux positions différentes du poisson suivi dans le temps et une deuxième ligne qui relie l'une des deux positions différentes et une position du transducteur.

6. Dispositif de calcul de taille de cible selon l'une quelconque des revendications 1 à 4, dans lequel :
le module de calcul d'angle d'incidence d'ultrasons (26) calcule l'angle d'incidence d'ultrasons en calculant un angle formé entre une deuxième ligne qui relie une position du poisson suivi et le transducteur et une troisième ligne qui est une ligne verticale partant du transducteur (22) vers le fond de l'eau.

7. Dispositif de calcul de taille de cible selon l'une quelconque des revendications 1 à 4, dans lequel :
le module de calcul d'angle d'incidence d'ultrasons (26) calcule l'angle d'incidence d'ultrasons sur la base d'une première distance entre le transducteur et une première position du poisson suivi à un premier moment et d'une seconde distance entre le transducteur et une seconde position du poisson suivi à un second moment différent du premier moment ; dans lequel :
le module de calcul d'angle d'incidence d'ultrasons (26) calcule l'angle d'incidence d'ultrasons sur la base d'une hypothèse que les première et seconde distances varient dans le temps sur la base d'une courbe hyperbolique.

8. Dispositif de calcul de taille de cible selon l'une quelconque des revendications précédentes, dans lequel :
la seconde partie corporelle est une surface extérieure du poisson suivi.

9. Procédé de calcul de taille de cible, comprenant les étapes consistant à :

émettre une onde d'émission vers des cibles sous-marines et générer un signal d'écho à partir d'une réflexion de l'onde d'émission sur les cibles sous-marines, au moyen d'un transducteur ;

suivre dans le temps un poisson parmi les cibles sous-marines à partir de signaux d'écho résultant de différentes ondes d'émission ;

calculer, pour chaque position du poisson suivi dans le temps, un angle d'incidence d'ultrasons de l'onde d'émission sur le poisson suivi, sur la base des signaux d'écho ;

calculer, pour chaque position du poisson suivi dans le temps, une distance entre une vessie natatoire du poisson suivi et une seconde partie corporelle du poisson suivi, différente de la vessie natatoire, à partir des signaux d'écho du poisson suivi ;

calculer un poids du poisson suivi sur la base de l'angle d'incidence d'ultrasons et de la distance calculée ; et

calculer un histogramme de l'angle d'incidence d'ultrasons en fonction de la distance à partir de l'angle d'incidence d'ultrasons et de la distance, calculés pour une pluralité de poissons suivis.

10. Support non temporaire lisible par ordinateur sur lequel sont stockées des instructions exécutables par ordinateur qui, lorsqu'elles sont exécutées par le dispositif selon la revendication 1, amènent le dispositif à exécuter le procédé selon la revendication 9.

11. Procédé de calcul de taille de cible, comprenant les étapes consistant à :

émettre une onde d'émission vers des cibles sous-marines et générer un signal d'écho à partir d'une réflexion de l'onde d'émission sur les cibles sous-marines, au moyen d'un transducteur ;

suivre dans le temps un poisson parmi les cibles sous-marines à partir de signaux d'écho résultant de différentes ondes d'émission ;

calculer, pour chaque position du poisson suivi dans le temps, un angle d'incidence d'ultrasons de l'onde d'émission sur le poisson suivi, sur la base des signaux d'écho ;

calculer, pour chaque position du poisson suivi dans le temps, une distance entre une vessie natatoire du poisson suivi et une seconde partie corporelle du poisson suivi, différente de la vessie natatoire, à partir des signaux d'écho du poisson suivi ; et

calculer un poids du poisson suivi sur la base de l'angle d'incidence d'ultrasons et de la distance calculée ;

le poids du poisson suivi étant calculé lorsqu'un premier angle d'incidence d'ultrasons de l'onde d'émission sur le poisson suivi à un premier moment et un second angle d'incidence d'ultrasons de l'onde d'émission sur le poisson suivi à un second moment différent du premier moment sont tels qu'un angle seuil donné se situe entre les premier et second angles d'incidence d'ultrasons, et le poids étant calculé sur la base de la distance du poisson suivi dans le premier angle d'incidence d'ultrasons et de la distance du poisson suivi dans le second angle d'incidence d'ultrasons.

12. Support non temporaire lisible par ordinateur sur lequel sont stockées des instructions exécutables par ordinateur qui, lorsqu'elles sont exécutées par le dispositif selon la revendication 4, amènent le dispositif à exécuter le procédé selon la revendication 11.

FIG. 1A

FIG. 1B

200

Signal Processing Unit 24

Ultrasound Incidence Angle Calculation Module 26

Fish Tracking Module 25

Distance Calculation Module 27

Ultrasound Incidence Angle-Distance Histogram Calculation Module 28

Peak Extraction Module 29

Fish Weight Calculation Module 30

Transceiver Unit 23

Transducer 22

FIG. 2

FIG. 3

FIG. 4

FIG. 5A

Tracked Fish @ $T_{n-1}$

Tracked Fish @ $T_n$

FIG. 5B

503

22

θ

504

500

505

Tracked Fish @ $T_{n-1}$

Tracked Fish @ $T_n$

FIG. 5C

FIG. 6

FIG. 7A

FIG. 7B

FIG. 8

FIG. 9

FIG. 10

FIG. 11A

FIG. 11B

FIG. 12

1300

```
            ┌─────────┐
            │  START  │
            └─────────┘
                 │
                 ▼
┌────────────────────────────────────────────────┐
│ Transmit a transmission wave toward underwater  │     1302
│ targets and generate an echo signal from a      │  ⌒
│ reflection of the transmission wave on the      │
│ underwater targets using a transducer           │
└────────────────────────────────────────────────┘
                 │
                 ▼
┌────────────────────────────────────────────────┐
│ Track in time a fish among the underwater       │     1304
│ targets from echo signals resulting from        │  ⌒
│ different transmission waves                     │
└────────────────────────────────────────────────┘
                 │
                 ▼
┌────────────────────────────────────────────────┐
│ Calculate for each position of the tracked fish │     1306
│ in time, an ultrasound incidence angle of the   │  ⌒
│ transmission wave on the tracked fish based on  │
│ the echo signals                                │
└────────────────────────────────────────────────┘
                 │
                 ▼
┌────────────────────────────────────────────────┐
│ Calculate for each position of the tracked fish │
│ in time, a distance between a swimbladder of    │     1308
│ the tracked fish and a second body part of the  │  ⌒
│ tracked fish, different from the swimbladder,    │
│ from the echo signals of the tracked fish       │
└────────────────────────────────────────────────┘
                 │
                 ▼
┌────────────────────────────────────────────────┐
│ Calculate a weight of the tracked fish based on │     1310
│ the ultrasound incidence angle and the          │  ⌒
│ calculated distance                             │
└────────────────────────────────────────────────┘
                 │
                 ▼
            ┌─────────┐
            │  STOP   │
            └─────────┘
```

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017163904 A1

- WO 2020090287 A

### Non-patent literature cited in the description

- Hydroacoustics for the discovery and quantification of Nassau grouper spawning aggregations. *Coral Reefs*, 2017, vol. 36 (2)